# EUROPEAN PATENT APPLICATION

(11) **EP 4 851 912 A1**
(43) Date of publication of application: **09.12.2026**
(21) Application number: 25305795.4
(22) Date of filing: 02.06.2025
(51) Int. Cl.: G06T 3/147, G06T 7/00, G06T 7/215

(54) **TRACKING A LESION OF A PATIENT**

(71) Applicant: Dassault Systèmes, 78140 Vélizy-Villacoublay (FR)
(72) Inventor: BORNACHOT, Yohan, 78946 Vélizy-Villacoublay (FR)
(74) Representative: Bandpay & Greuter

(57) **Abstract**

The disclosure notably relates to a computer implemented method for tracking a lesion from at least two sets of images representing the lesion. The method comprises obtaining a baseline set of images, and at least one annotation related to the lesion represented by at least one image of the set, being associated to a bounding box. The method also comprises obtaining a follow-up set of images, performing a registration of the baseline set and the follow-up set, the registration thereby obtaining a transformation that aligns the baseline set and the follow-up set, applying the obtained transformation on the at least one annotation, thereby obtaining the transformed bounding box in the follow-up set, and determining images in the follow-up set enclosed by the transformed bounding box and segmenting a lesion from the determined images, the lesion in the determined images enclosed by the transformed bounding box representing the tracked lesion.

## Description

### TECHNICAL FIELD

The disclosure relates to the field of computer programs and systems, and more specifically to a method, system and program for tracking a lesion of a patient from at least two sets of images representing the lesion.

### BACKGROUND

Lesions refer to abnormal changes in human body tissue that can indicate various conditions, such as tumors, inflammation, or injury. There is a need to follow the evolution of lesions and assess treatment response by longitudinal lesions tracking. Such tracking is of high importance for cancer management in clinical practice. When the radiologist does lesion matching across different time points manually, it can be time consuming and represents a prohibitive amount of work.

Within this context, there is still a need for an improved method for tracking a lesion of a patient from at least two sets of images representing the lesion.

### SUMMARY

It is therefore provided a computer implemented method for tracking a lesion of a patient from at least two sets of images representing the lesion. Each set of images is acquired at different time points. The method comprises obtaining a baseline set of images. The method also comprises obtaining at least one annotation related to the lesion represented by at least one image of the set. The at least one annotation is associated to a bounding box. The method also comprises obtaining a follow-up set of images. The method also comprises performing a registration of the baseline set and the follow-up set. The registration thereby obtains a transformation that aligns the baseline set and the follow-up set in space. The method also comprises applying the obtained transformation on the at least one annotation. The method thereby obtains the transformed bounding box in the follow-up set. The method also comprises determining images in the follow-up set enclosed by the transformed bounding box. The method also comprises segmenting a lesion from the determined images. The lesion in the determined images enclosed by the transformed bounding box represents the tracked lesion from the baseline set.

The method may comprise one or more of the following:
- further comprises, after applying (S40) the obtained transformation and prior to determining images in the follow-up set enclosed by the transformed bounding box and segmenting the lesion in the determined images, increasing the dimension of the bounding box;
- the obtained transformation is one of an affine transformation or a deformable registration;
- the obtained transformation is an affine transformation of the type: $transform = \left(\begin{matrix}{α}_{x} & {r}_{xy} & {r}_{xz} \\ {r}_{yx} & {α}_{y} & {r}_{yz} \\ {r}_{zx} & {r}_{zy} & {α}_{z}\end{matrix}\right) ⋅ \left(\begin{matrix}x \\ y \\ z\end{matrix}\right) + \left(\begin{matrix}{C}_{x} \\ {C}_{y} \\ Δ z\end{matrix}\right) = A . X + T$ wherein ***αₓ, α_{y}, α_{z}*** denote dilation factors in [*x,y,z*] coordinates, *r_{xy}, r_{xz}, r_{yx}, r_{yz}, r_{zx}, r_{zy} denote rotation coef ficients,* C*ₓ,* C*_{y}* denote translation components in the x and y coordinates, and Δz denotes a vertical translation offset in z coordinates
- the bounding box is a 2D bounding box, and segmenting the lesion in the determined images comprises obtaining a neural network configured to take as input a set of images and to output a 2D segmentation of a lesion from the set of images, and applying the neural network to the determined images; or the bounding box is a 3D bounding box, and segmenting the lesion in the determined images comprises obtaining a 3D segmentation of the lesion;
- further comprises inferring a new lesion in the follow-up set by:
   o performing a segmentation of the baseline set of images; and
   o comparing the segmentation in the determined images with the segmentation of the baseline set of images;
- performing the segmentation of the baseline set of images comprises applying a trained segmentation neural network to the baseline set, the trained segmentation neural network being configured to take as input one or more images and at least one annotation associated to a bounding box and to output a pair of a respective segmentation of a lesion and a respective bounding box enclosing the lesion;
- performing the registration of the baseline set and the follow-up set comprises aligning the baseline set and the follow-up set along a common reference axis, the common reference axis being normal to each image of the baseline set and the follow-up set, the alignment being performed by:
   o obtaining a predetermined interslice space for each of the baseline set and the follow-up set;
   o computing, for each set, a set of coordinates lying in a scale common to the baseline set and the follow-up set each coordinate being associated, in the common reference axis, with an image;
   o computing an intersection between the two sets of coordinates, thereby identifying overlapping images, which having coordinates which lie in the intersection;
   o creating, for each set, a new physical scale of coordinates sharing a common origin, and wherein two consecutive coordinates of the new physical scale being are spaced by the predetermined interslice space;
   o computing, for each set, the mean of the new physical scale of coordinates associated to overlapping slices;
   o subtracting the mean computed for the follow-up set to the mean computed for the baseline set, thereby obtaining a vertical offset along the common reference axis; and
   o aligning the baseline set and the follow-up set by offsetting the follow up set with the obtained vertical offset;
- computing the set of coordinates comprises:
   o providing another neural network, the other neural network being configured to take as input a pair of sets of images and to output a set of coordinates lying in a scale common to the pair of sets, each coordinate being associated, in the common reference axis, with an image;
   o inputting the baseline set and the follow-up set to the other neural network; and
   ∘ outputting the set of coordinates from the other neural network;
- the other neural network is trained according to a machine-learning method comprising:
   o providing a dataset of sets of images; and
   o training the other neural network based on the dataset, the training comprising providing as input a pair of sets of images from the dataset and regressing a slice score for each image of the set;
- each image is a CT-Scan; and/or
- further comprises displaying and/or storing the aligned baseline set and follow-up set of images.

It is further provided a computer program comprising instructions for performing the method.

It is further provided a computer readable storage medium having recorded thereon the computer program.

It is further provided a system comprising a processor coupled to a memory, the memory having recorded thereon the computer program.

### BRIEF DESCRIPTION OF THE DRAWINGS

Non-limiting examples will now be described in reference to the accompanying drawings, where:
- FIG. 1 shows a flowchart of an example of the method;
- FIG. 2 shows an example of the system;
- FIG.s 3 to 7 illustrate the method.

### DETAILED DESCRIPTION

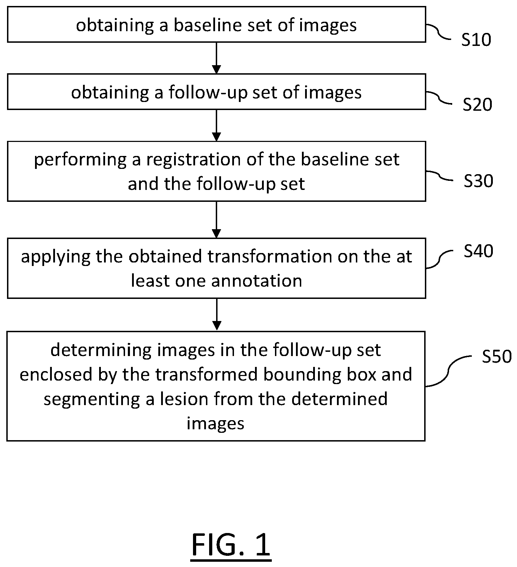

With reference to the flowchart of FIG. 1, it is proposed a computer implemented method for tracking a lesion of a patient from at least two sets of images. The images represent the lesion. Each set of images is acquired at different time points. The method comprises obtaining S10 a baseline set of images. The method also comprises obtaining at least one annotation related to the lesion represented by at least one image of the set. The at least one annotation is associated to a bounding box. The method also comprises obtaining S20 a follow-up set of images. The method also comprises performing S30 a registration of the baseline set and the follow-up set. The registration thereby obtains a transformation that aligns the baseline set and the follow-up set in space. The method also comprises applying S40 the obtained transformation on the at least one annotation. The method thereby obtains the transformed bounding box in the follow-up set. The method also comprises determining S50 images in the follow-up set enclosed by the transformed bounding box. The method also comprises segmenting a lesion from the determined images. The lesion in the determined images enclosed by the transformed bounding box represents the tracked lesion from the baseline set.

The invention thus allows to propagate baseline lesion information performed during a medical exam to a follow up exam, allowing the practitioner to have all information available at glance, as the segmentation enables an accurate lesion matching. Indeed, the method enables to identify variations in physical space represented by respective images of the baseline set and the follow-up set, as the segmented lesion on the follow-up set represent the evolution of the tracked lesion as represented by the baseline set.

Moreover, the method achieves a good balance between the speed for performing the transformation and accuracy. This is all thanks to the method propagating spatial information (in the form of the lesion segmented from the determined images) by looking for lesions inside the transformed bounding boxes. Furthermore, the method guarantees complete lesion matching management.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

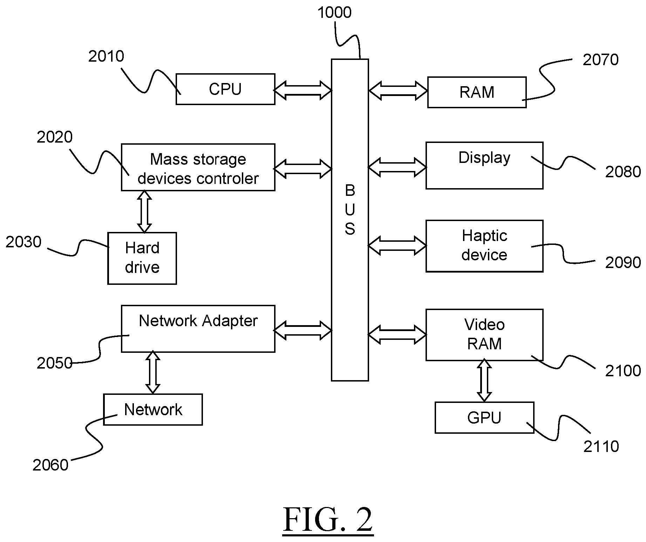

FIG. 2 shows an example of the system, wherein the system is a client computer system, e.g. a workstation of a user.

The client computer of the example comprises a central processing unit (CPU) 2010 connected to an internal communication BUS 2000, a random access memory (RAM) 2070 also connected to the BUS. The client computer may be further provided with a graphical processing unit (GPU) 2110 which is associated with a video random access memory 2100 connected to the BUS. Video RAM 2100 is also known in the art as frame buffer. The client computer may be further provided with a mass storage device controller 2020 manages accesses to a mass memory device, such as hard drive 2030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 2050 manages accesses to a network 2060. The client computer may also include a haptic device 2090 such as cursor control device, a keyboard or the like. A cursor control device is used in the client computer to permit the user to selectively position a cursor at any desired location on display 2080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

The method comprises obtaining S10 the baseline set of images and obtaining S20 the follow-up set of images.

Obtaining S10 the baseline set of images and obtaining S20 the follow-up set of images may comprise retrieving such sets from a non-volatile storage, or retrieving them from a network. The baseline set of images and the follow-up set of images may be obtained at different time points, by different persons/entities. The follow-up set of images may be obtained at a time after obtaining S10 the baseline set of images. In other words, the obtention S10 of the baseline set of images is performed at a time preceding the obtention S20 of the follow-up set of images.

The images of the baseline set and the follow-up set represent the lesion. As known in the art, the baseline set refer to the initial set of medical images taken of the lesion, e.g. at the starting point of observation or treatment. As known in the art, the follow-up set of images are subsequent medical images taken after the baseline images, at later time points, to monitor the evolution or response of a lesion, condition, or treatment over time. The images of the baseline set may sample a spatial volume enclosing a portion of the human body comprising the lesion. Respectively, the images of the follow-up set also sample a spatial volume enclosing a portion of the human body comprising the lesion at the time after obtaining the baseline set. Accordingly, the follow-up set encloses a spatial volume containing variations in the morphology of the portion of the human body, e.g., including a temporal evolution of the lesion. The slices of the follow-up set of images may stem from a medical imaging performed during a follow up procedure of the patient, e.g., at a time period (e.g., days or months) after the baseline set of images was obtained.

In the context of the present disclosure, a lesion may be a group of cells, e.g., in close proximity, a portion of tissue of the body of the patient and subject to a physiological condition. In other words, the lesion may be a portion of tissue of the body of the patient subject to an anomaly, without presuming the pathogenicity in the tissue. In examples, in the context of oncology the term lesion may be used for referring to a tumor. The tissue may correspond to an organ (such as the liver, lungs, heart or bones) or may correspond to a portion of the organ.

The baseline set and/or the follow-up set of images obtained at S10 may be ordered according to a reference axis. Each image may comprise a representation of the lesion viewed from a view at a position of a reference axis, e.g., orthogonal with respect to the reference axis. The reference axis may be set by convention, e.g., a standard z axis along the human body portion of the patient comprising the lesion, in which case each view may comprise a view on a plane orthogonal to the reference axis, for example the standard x-y plane with respect to the z axis, also called an axial transversal view of the lesion. Alternatively, the view may be for example a sagittal (also called longitudinal) view or frontal (also called coronal) view. The images of the baseline set and/or the follow-up set may comprise information of the position of the images with respect to the reference axis, that is, the position of the reference axis in which the representation of the lesion is viewed. The images of the baseline set and/or the follow-up set may be ordered according to such information. In examples, the images of the baseline set and/or the follow-up set may be evenly distributed, that is, the images of the baseline set (respectively the follow-up set) sample the human body portion (with a same separation between two consecutive images) along the reference axis. For example, the images may be separated along the reference axis by 1mm or more.

The method also comprises obtaining at S10 at least one annotation related to the lesion represented by at least one image of the baseline set. By annotation it is meant any kind of information on the lesion represented by the at least one image. The at least one annotation is associated to a bounding box. As known in the art, annotating means labeling or marking one or more relevant features in at least one image of the baseline set., e.g. labeling regions by assigning a class or diagnosis to certain areas (e.g., "benign tumor", "malignant", "edema"), adding metadata - such as measurements, position, or clinical notes. The annotation may be automatically performed, e.g. by using a machine learning method, or it may be done by an expert such as radiologists or clinicians. It is to be understood that the annotation may be also referred to as volumetric annotation as it is associated to a bounding box.

By "bounding box" it is meant any geometrical shape in any dimension such as two or more, e.g., three dimensions, for example a, two-dimensional rectangle (also called "2D bounding box") in two dimensions or a rectangular parallelepiped in three dimensions. In the case of three dimensions, the bounding box may also be called a bounding volume or a 3D bounding box. In the case of the 2D bounding box, the 2D bounding box may enclose a 2D region inside the at least one image. In the case of a plurality of images representing the lesion, each image may be related to an annotation associated to a respective 2D bounding box. The bounding box may enclose the lesion. In other words, the bounding box has a location and dimension in (three-dimensional) space so as to enclose the location in space of the lesion as represented by the at least one image. In the case of the 2D bounding box, the 2D bounding box may enclose pixels of the at least one image indicative of the presence of the lesion. The method may apply standard conversions from the space sampled by the at least one image and the space of the bounding box. In examples, the 3D bounding box may enclose each portion of the lesion represented by multiple consecutive images representing a portion of the body of the patient.

In examples, each image of the baseline set and/or the follow-up set of images obtained at S10 may be a CT-Scan. A CT-scan (Computed Tomography Scan) is a specific type of medical images produced by sending X-rays through the human body. The signal is read and analyzed to reconstruct a dense volume of the body. Practically, the volume is split into slices whose normal direction (set as the standard z-axis) is feet to head. Alternatively, each image may be an MRI, a PET Scan or SPECT Scan. An MRI (Magnetic Resonance Imaging) is a specific type of medical images technique that uses a magnetic field and computer-generated radio waves to create detailed images of the organs and tissues in the body. PET-scan (Positron Emission Tomography) may be an imaging test that can help reveal the metabolic or biochemical function of your tissues and organs. The PET scan may use a radioactive drug called a tracer to show both typical and atypical metabolic activity. A SPECT Scan may be the result of a type of nuclear imaging test that provides 3D images of internal organs and tissues. SPECT-Sanning may use a Gamma camera and a radioactive tracer to evaluate how blood flows to tissues and organs, as well as to assess their function.

In examples, obtaining S10 the baseline set and/or the follow-up set of images may be based on performing a medical imaging on a patient. In other words, the baseline set and/or the follow-up set of images may each stem from a medical imaging performed on an anatomical region of a patient enclosing a portion of the human body tissue comprising the lesion at a given time. The medical imaging may be, for example, CT-Scanning, Medical Resonance Imaging - MRI, Positron Emission Tomography -PET Scanning or Single photon emission computed tomography - SPECT scanning.

Back to FIG. 1, the method comprises performing the registration S30 of the baseline set and the follow-up set. Registration in the context of the technical field of the present disclosure refers to the process of spatially transforming different sets of data into one common coordinate system. Said otherwise, the registration is the process of transforming different sets of images into a common coordinate system, so that they can be compared accurately. In other words, images of the baseline set and the follow-up set are aligned spatially so as to enable comparison across different time points in the common coordinate system. Performing the registration S30 may include, e.g., applying rigid transformations (including e.g., rotations, translations and/or reflections) or deformable (also called "non-rigid" and including, e.g., compressions or expansions) transformations into the images of the baseline set.

The registration S30 thereby obtains a transformation that aligns the baseline set and the follow-up set. In other words, the registration obtains a mapping from the baseline set to the follow-up set that compensates differences, e.g., in positioning orientation and/or deformations between the images of the baseline set and the images of the follow-up set.

Examples of the transformation resulting from the registration are now discussed.

In examples, the obtained transformation may be one of an affine transformation or a deformable registration. As known in the art, the affine transformation is a geometric transformation that preserves lines and parallelism, that is, applying rigid transformations. The deformable transformation (also called "displacement field") may apply compressions or expansions.

In examples, the registration may take as input an initialization transformation of the type: $init transform = \left(\begin{matrix}{α}_{x} & 0 & 0 \\ 0 & {α}_{y} & 0 \\ 0 & 0 & {α}_{z}\end{matrix}\right) ⋅ \left(\begin{matrix}x \\ y \\ z\end{matrix}\right) + \left(\begin{matrix}{C}_{x} \\ {C}_{y} \\ Δ z\end{matrix}\right) = A . X + T .$

***αₓ, α_{y}, α_{z}*** denote dilation factors in [*x,y,z*] coordinates. Each of the dilation factors correspond to pixel spacing ratios between both CT-scan along each dimension (∀*d* ∈ [*x,y,z*]*,* ${α}_{i} = \frac{pixel {spacing}_{BL , d}}{pixel {spacing}_{FU , d}}$). C*ₓ,* C*_{y}* denote translation components in the x and y coordinates, i.e., 2D translation components. Δz denotes a vertical translation offset in z coordinates.

The method allows to infer the vertical offset z between volumes (rather than the center aligning vertical offset). Dilation factors ***αₓ, α_{y}, α_{z}*** are set to the ratio of pixel spacings, which gives a good approximation for the final parameters. This improves final registration, which may be sensitive to initialization.

The obtained transformation resulting from the registration is of the type: $transform = \left(\begin{matrix}{α}_{x} & {r}_{xy} & {r}_{xz} \\ {r}_{yx} & {α}_{y} & {r}_{yz} \\ {r}_{zx} & {r}_{zy} & {α}_{z}\end{matrix}\right) ⋅ \left(\begin{matrix}x \\ y \\ z\end{matrix}\right) + \left(\begin{matrix}{C}_{x} \\ {C}_{y} \\ Δ z\end{matrix}\right) = A . X + T$

***αₓ, α_{y}, α_{z}*** denote dilation factors in [*x,y,z*] coordinates. Each of the dilation factors correspond to pixel spacing ratios between both CT-scan along each dimension (∀*d* ∈ [*x,y,z*]*,* ${α}_{i} = \frac{pixel {spacing}_{BL , d}}{pixel {spacing}_{FU , d}}$). *r_{xy}, r_{xz}, r_{yx}, r_{yz}, r_{zx}, r_{zy} denote rotation coefficients.* C*ₓ,* C*_{y}* denote translation components in the x and y coordinates, i.e., 2D translation components. Δz denote a vertical translation offset in z coordinates.

The dilation factors may control uniform or non-uniform scaling along different axes to account for size variations between the baseline set and follow-up set. The translation components may control the shifting the follow-up set relative to the baseline set to achieve spatial alignment. The vertical translation offset may control a shift in z coordinates to accommodate differences in vertical positioning of the baseline set relative to the follow-up set.

The method also comprises applying S40 the obtained transformation on the at least one annotation. In other words, the transformation is also propagated to the at least one annotation associated to the bounding box. The method thereby obtains the transformed bounding box in the follow-up set. In other words, the method thereby adjusts the spatial location and dimensions (e.g., 2D or 3D) of the bounding box into space sampled by the follow-up set, taking into account the transformation obtained at S30.

The method also comprises determining S50 images in the follow-up set enclosed by the transformed bounding box and segmenting a lesion from the determined images. The determination S50 may comprise obtaining information on the location in 3D space associated to the images of the follow-up set, and determine the intersection of the images with the bounding box after being transformed. The lesion represented by the determined images enclosed by the transformed bounding box thereby represents the tracked lesion from the baseline set.

The method thus improves tracking a lesion of a patient from at least two sets of images representing the lesion.

Indeed, the method enables the propagation of baseline information on the lesion obtained from the baseline set (e.g., as represented by the bounding box, segmentation and other information such as diameter measures of the lesion) to the follow-up set. In other words, the method guarantees complete matching lesion management in the sense that each identified follow-up lesion is related to a counterpart represented in the baseline set, while also taking into account possible new lesions that only appear in the follow-up set. The method thus obtains complete 3D information about the temporal evolution of the lesion after the baseline set was obtained. The method consequently allows the practitioner to perform (e.g., exact) lesion matching from the available information, which enables the practitioner making medical follow-up decisions.

This is all thanks to the specific steps applied by the method. Specifically, the registration S30 performed on the baseline set and the follow-up set allows to align the baseline set and the follow-up set in space. Thus, the method compensates misalignments due to variations in the image acquisition setup, positioning of the patient or different sampling. Hence, the alignment obtained by the transformation provides an efficient initialization prior to spatially transforming the baseline set and the follow-up set into one common coordinate system. Indeed, knowing the difference in alignment, for example due to a vertical offset, is a better initialization than, e.g., sampling random parameters or aligning volume centers for initializing the transformation, e.g., before an optimization process.

The application S40 of the obtained transformation propagates the bounding box information obtained from the at least one annotation related to the lesion from the space defined by the alignment of the baseline set and the follow-up set. In other words, the bounding box on the baseline set is warped so as to occupy the same space (and hence cover the same lesion) in the follow-up set.

The determination S50 of images in the follow-up set enclosed by the transformed bounding box enable the identification of lesions tracked from the baseline set. Consequently, a medical practitioner may perform visual comparisons of the lesions represented in the follow-up set compared to their corresponding baseline set so as to determine the evolution of the lesions, e.g., aided by a matching. Thus, the medical practitioner may perform a decision on the patient based on the evolution of the lesion.

In examples, the method may further comprise displaying the aligned baseline set and follow-up set of images. The method may comprise rendering the aligned images of each set on a display device. The displaying thereby enables the comparison of the aligned sets. The method may further comprise user-interaction when displaying the baseline set and follow-up set of images. The user-interaction may comprise for example zooming, panning, or changing the color of the images of each set. The method thus enhances user analysis for diagnostic, analytical, or treatment planning purposes. The displaying may further comprise superposing the aligned baseline set and follow-up set of images. For example, the images of the baseline set may be opaque and the follow-up set may have a degree of transparency, e.g. 50% transparency, or vice versa. The method may further comprise displaying the determined images of the follow-up enclosed by the transformed bounding box. The display may comprise setting another degree of transparency or highlight compared to other images of the follow-up set.

The method may further comprise storing the aligned baseline set and follow-up set of images. The baseline set and follow-up set may be stored in a digital format, e.g., in a non-volatile storage, a database or a cloud-based storage system. The storage may include metadata associated with the alignment process, such as timestamps.

The method may thereby enable the analysis of the evolution of the lesion in the follow-up set by comparing the differences between the baseline set and follow-up set of images, thereby improving decision making in a medical context.

In examples, the method may further comprise, after applying S40 the obtained transformation and prior to determining images in the follow-up set enclosed by the transformed bounding box and segmenting the lesion in the determined images, increasing the dimension of the bounding box. For example, when the bounding box is a 2D bounding box, the method may comprise increasing the area in a corresponding image comprising the 2D bounding box. When the bounding box is a 3D bounding box, the method may comprise increasing the volume enclosed by the 3D bounding box. The increase of the dimension may take into account, e.g., variations in the lesion represented in the follow-up set with respect to the baseline set, for example variations in positioning, shape, or size. The variations may be due, e.g., to inconsistencies in imaging acquisition such as the movement of the patient, or to biological changes over time. The increase of the dimension may be applied uniformly in all directions (i.e., on all faces of the bounding box). Alternatively, the increase of the dimension may be based on predefined scaling factors (e.g., twice the original size or more), adaptive thresholds, or anatomical considerations.

In examples, a lesion represented by the baseline set, the method comprises transforming 3D bounding box coordinates into the space defined by the follow-up set, defining standard (x, y, z) coordinates. The method may comprise straightening the bounding box, so as to take into account rotation, by taking extremal coordinates of the transformed 3D bounding box along each dimension. The z coordinates (*zₘᵢₙ*, *zₘₐₓ*) of the transformed 3D bounding box define the range of images of the follow-up set that are related to the lesion represented by the baseline set. That is, images associated to a z coordinate in the follow-up set for which the associated z-coordinate is comprised in the interval [*zₘᵢₙ*, *zₘₐₓ*] are candidate images representing the lesion in the follow-up set.

For each candidate image, the method may comprise performing a 2D projection of the transformed 3D bounding box. The method may comprise increasing the dimension of the 2D projection, e.g., based on the radius of the lesion represented by the corresponding image of the baseline set. The 2D projection may define the searching area for further segmentation. The increase of the dimension allows to increase the searching area because the lesion has possibly moved in the body or changed in size under treatment. In addition, the increase of the dimension may compensate some registration errors. The choice of the radius of the baseline lesion to enlarge the bounding box size is arbitrary and is used to adapt the target bounding box size to the lesion size: it considers that the lesion cannot grow more than twice its original size between baseline and follow-up exam, or that it cannot move more than its radius inside the body.

This results in an improved tracking of the lesion. Indeed, as the method increases the dimension of the bounding box, the method ensures that the transformed bounding box does not exclude parts of the lesion represented in images of the follow-up set. Thus, the method mitigates errors due to the evolution of the lesion or other variations that may change the dimension or location of the location of the lesion relative to the baseline set. For example, this allows to determine if the lesion has moved or increased in size and may compensate for registration errors, e.g., gaps found during registration.

In examples, the bounding box may be a 2D bounding box. In examples, the 2D bounding box may be a 2D projection of a 3D bounding box on a region of an image (e.g., of the baseline set and/or the follow-up set). Segmenting the lesion in the determined images may comprise obtaining a neural network. The neural network may be configured to take as input a set of images and to output a 2D segmentation of a lesion from the set of images. 2D segmentation refers to grouping pixels that belong to a same object by categorizing each pixel value of an image to a particular class, as known in the art. The neural network may be a function comprising a collection of connected nodes, also called "neurons". Each neuron receives an input and outputs a result to other neurons connected to it. The neurons and the connections linking each of them have weight values, which are adjusted via a training. For example, the neural network may be a deep convolutional network based on a Mask R-CNN neural network, such disclosed in Yan, K., Tang, Y., Peng, Y., Sandfort, V., Bagheri, M., Lu, Z., & Summers, R. M. (2019), "MULAN: multitask universal lesion analysis network for joint lesion detection, tagging, and segmentation", Medical Image Computing and Computer Assisted Intervention-MICCAI 2019: 22nd International Conference, Shenzhen, China, October 13-17, 2019*,* the entire contents being incorporated by reference.

Segmenting the lesion may comprise applying the neural network to the determined images (of the follow-up set). In other words, the output of the neural network may be a segmentation, i.e., a group of pixels indicative of the presence of the lesion in the image of the follow-up image. The segmentation may be a segmentation mask.

A mask may be a binary image consisting of zero and non-zero values. Pixels corresponding to the segment (representing the lesion) may have a predetermined non-zero value, e.g., one, whereas other pixels (for instance, pixels corresponding to other portions of the human body) have a value of zero.

In examples, when the bounding box is a 3D bounding box, segmenting the lesion in the determined images may comprise obtaining a 3D segmentation of the lesion. In other words, the method comprises obtaining a volumetric region in 3D space identifying the lesion. The determination of the 3D bounding box may be performed with a neural network configured to take as input a set of images and output a 3D segmentation, e.g., based on 3D-UNET as disclosed in Asnawi, M. H., Pravitasari, A. A., Darmawan, G., Hendrawati, T., Yulita, I. N., Suprijadi, J., & Nugraha, F. A. L., "Lung and Infection CT-Scan-Based Segmentation with 3D UNet Architecture and Its Modification", In Healthcare (Vol. 11, No. 2, p. 213*). MDPI,* or SwinUNETR as disclosed in Yazdani, E., Karamzadeh-Ziarati, N., Cheshmi, S. S., Sadeghi, M., Geramifar, P., Vosoughi, H., ... & Kheradpisheh, S. R.. "Automated segmentation of lesions and organs at risk on [68Ga] Ga-PSMA-11 PET/CT images using self-supervised learning with Swin UNETR", Cancer Imaging, 24(1), 30*.*

The method thus achieves a good balance in execution speed and accuracy of the detection of the lesion. Indeed, neural networks have been noticed to be fast while maintaining accuracy.

In examples, the method may further comprise inferring a new lesion in the follow-up set. Inferring the new lesion may comprise performing a segmentation of the baseline set of images. In other words, the inference may comprise obtaining one or more segments representative of a lesion on the images of the baseline set. The inference may comprise applying a neural network as above.

Inferring the new lesion may also comprise comparing the segmentation in the determined images with the segmentation of the baseline set of images. The comparison may be performed with, e.g., pixel per pixel comparison, or applying an overlapping region-based matching algorithm. This enables the identification of new lesions that may have appeared in the time after obtaining the baseline set and before obtaining the follow-up exam.

In examples, performing the segmentation of the baseline set of images may comprise applying a trained segmentation neural network to the baseline set. The trained segmentation neural network being configured to take as input one or more images. The trained segmentation neural network may also be configured to take as input at least one annotation associated to a bounding box. The trained segmentation neural network may also be configured to output a pair of a respective segmentation of a lesion and a respective bounding box enclosing the lesion.

In the context of the present disclosure, by "trained segmentation neural network", it is meant that weights of the neural network are definitively saved after performing a learning/training on a dataset. The trained neural network may be a deep convolutional neural network such as a Mask R-CNN neural network set forth in the document Kaiming He, Georgia Gkioxari, Piotr Dollár, Ross Girshick Mask R-CNN, arXiv:1703.06870*.* The network may have been trained using a dataset of annotated medical images, leveraging supervised learning techniques with manually or semi-automatically labeled ground truth data. The dataset may comprise medical images from the DeepLesion dataset, for example as set forth in the document Yan K, Wang X, Lu L, Summers RM, DeepLesion: automated mining of large-scale lesion annotations and universal lesion detection with deep learning, J Med Imaging (Bellingham), 2018*.*

In examples, the trained segmentation neural network may be as in the European patent application EP23307178.6, the entire contents being incorporated herein by reference.

Performing S30 the registration of the baseline set and the follow-up set may comprise aligning the baseline set and the follow-up set along a common reference axis.

In other words, images of the baseline set and the follow-up set are arranged so as to match (approximately or substantially) spatially in the common reference axis. For example, a respective image of the baseline set and a respective image of the follow-up set may represent a same spatial location in the common reference axis.

The common reference axis may be a reference line in a coordinate frame (e.g., a Cartesian coordinate frame) for locating coordinates for images of the baseline set and the follow-up set. The common reference axis may be based on anatomical information, e.g., based on the length of the human body when viewed from an anterior view (in other words, a frontal view). The common reference axis may be normal to each image of the baseline set and the follow-up set. For example, the common reference axis may extend from a location at a lower portion of the body (e.g., the legs of the body) to a location at an upper portion of the body (e.g., the chest, neck or head). The common reference axis may be oriented, e.g., from the location at the lower portion of the body to the location at the upper portion of the body. The orientation may be set according to any convention. For example, the common reference axis may have a value of zero at the location at the lower portion of the body and a value of one at the location of the upper portion of the body. The common reference axis is normal to each image of baseline set and the follow-up set. In other words, coordinates associated to each image of the baseline set and the follow-up set may have values between zero and one.

The alignment may be performed by obtaining a predetermined interslice space for each of the baseline set and the follow-up set. By "interslice space" it is meant the distance by which two consecutive images (as found in the -ordered-baseline or follow-up set) are separated over the portion of the human body tissue that is represented by each image. The predetermined interslice space may be uniform, i.e., two consecutive images of a set (among the baseline set or the follow-up set) are separated by the same distance, e.g., along a predetermined axis, such as the standard z-axis. Alternatively, the predetermined interslice space may be not uniform, which case two consecutive images of a set may be separated by different distances.

In examples, the first and second sets are of CT-scans. Each of the sets may be ordered vertically, that is, from the lower portion of the body (e.g., the feet) to an upper part of the body (e.g., the head). The direction from the lower portion of the body to the upper part of the body may be set as the standard z axis. In other words, each CT-scan is a medical image that refers to an anatomical location in human body, ordered vertically. In examples, the CT-scans may be associated to CT-scan file metadata. The CT-scan file metadata may comprise axial coordinates corresponding to each image. The predetermined interslice space may be distance between axial coordinates (in terms of the axial axis) of two consecutive images.

The alignment may also comprise computing, for each set, a set of coordinates lying in a scale common to the baseline set and the follow-up set each coordinate being associated, in the common reference axis, with an image. In other words, the method assigns a coordinate for each image of a respective set. The common reference axis may be a standard axis, e.g., following a vertical axis, a horizontal axis or other anatomical axis such as an axis orthogonal to an axial view plane (similarly a sagittal view plane or a frontal view plane). In other words, the scale maps a respective image into a position relative to the common reference axis. The scale may have a predetermined spacing between two consecutive coordinates. In other words, the scale may be seen as setting a grid of units into which the common reference axis is divided. Each unit of the grid may be limited by two consecutive coordinates.

The alignment may also comprise computing an intersection between the two sets of coordinates. That is, the method identifies coordinates associated to images of the baseline set first and the follow-up set which are proximate along the common reference axis. The method thereby comprises identifying overlapping images having coordinates which lie in the intersection. In other words, the method identifies images from the baseline set and the follow-up set which enclose a volume that jointly represents the same anatomical region.

The alignment may also comprise creating, for each set, a new physical scale of coordinates. The new physical scale is a new mapping, for the baseline set and/or the follow-up set, for coordinates that takes into account the physical distribution of the images along the sampled portion of the human body. In other words, the predetermined spacing between two consecutive coordinates of the physical scales is separated with a distance based on the physical separation of the portion of the human body represented by the images of the respective baseline set or follow-up set. The new physical scale of coordinates, of each of the baseline set or the follow-up set, share a common origin. In other words, the origin of both new physical scale of coordinates overlap. Two consecutive coordinates of the new physical scales are spaced by the predetermined interslice space. In other words, the two consecutive coordinates of the new physical scales are spaced based on the physical separation set by the predetermined interslice space.

In the context of CT-Scan images, each image is a medical image that refers to an anatomical location in human body, ordered vertically. Axial coordinates corresponding to each image may be associated to CT-scan file metadata. However, the axial coordinates are relative and proper to their respective of the baseline set or the follow-up set. Based on this input, the alignment may comprise creating a common vertical scale that is shared by the baseline set and the follow-up set, thereby allowing the alignment of the baseline set and the follow-up set for visual comparison and medical follow up.

The alignment may also comprise computing, for each set, the mean of the new physical scales of coordinates associated to overlapping images. The mean of the new physical scales for each of the baseline set or the follow-up set may be the summation of coordinates in the set divided by the cardinality of the set of coordinates.

Computing, for each of the baseline set or the follow-up set, the new physical scale of coordinates sharing the common origin may comprise setting a respective set of new coordinates. In other words, the creation transforms the subset of coordinates of both sets which lie in the intersection into a new position on the common reference axis that takes into account the predetermined interslice space. A new coordinate of the respective set may correspond to the common origin added to an index of a respective image of the baseline set (respectively follow-up set) multiplied by an interslice space for the set.

Setting the respective set of coordinates may be performed, for a respective image of each set according to the formula: $∀ i ∈ \left[1, {N}_{1}\right] , {z}_{i} = {z}_{0} + i \times {δ}_{{z}_{1}}$ $∀ j ∈ \left[1, {N}_{2}\right] , {z}_{j} = {z}_{0} + j \times {δ}_{{z}_{2}}$ wherein *N*₁, denotes the number of images comprised in the baseline set. *N*₂ denotes the number of images comprised in the follow-up set. *i* denotes an index of an image of the baseline set. *j* denotes an index of an image of the follow-up set. *z*₀ denotes the common origin of the physical scale for each volume. δ_{z₁}, δ_{z₂} denote respectively an interslice space for the baseline set and the follow-up set.

Computing (S50) the mean of the new physical scale of coordinates for each set may be performed according to the formulae: $\overline{{Z}_{1}} = \frac{1}{Card \left({O}_{1}\right)} {\sum }_{{z}_{i} ∈ {O}_{1}} {z}_{i}$ $\overline{{Z}_{2}} = \frac{1}{Card \left({O}_{2}\right)} {\sum }_{{z}_{i} ∈ {O}_{2}} {z}_{i}$

*̅Z̅*̅₁̅ denotes the mean of the new scale of coordinates for the baseline set. Z̅₂̅ denotes the mean of the new scale of coordinates for the follow-up set. *O*₁ denotes the set of new coordinates for the new physical scale of coordinates for the baseline set and *O*₂ denotes the set of new coordinates for the new physical scale of coordinates for the follow-up set.

In other words, for the baseline set, *̅Z̅*̅₁̅ denotes the summation of coordinates in the set of coordinates *O*₁, denoted as ∑_{zᵢ} ∈ _{*O*₁} *zᵢ*, divided by the cardinality of the set of coordinates, denoted as *Card*(*O*₁)*.* For the follow-up set, Z̅₂̅ denotes the summation of coordinates in the set of coordinates *O*₂, denoted as ∑_{zᵢ} ∈ _{*O*₂} *zᵢ*, divided by the cardinality of the set of coordinates, denoted as *Card*(*O*₂)*.*

The alignment may also comprise subtracting the mean *̅Z̅*̅₁̅ of the new physical scale of coordinates for the follow-up set to the mean *̅Z̅*̅₂̅ of the new physical scale of coordinates computed for the baseline set. The method thereby comprises obtaining a vertical offset along the common reference axis. In other words, the vertical offset represents the spatial displacement along the new physical scale of coordinates.

Obtaining the vertical offset along the common reference axis may be performed according to the formula: $Δ {Z}_{2 \to 1} = \overline{{Z}_{1}} - \overline{{Z}_{2}} .$

Δ*Z*_{2→1} denotes the vertical offset. *̅Z̅*̅₁̅ denotes the mean of the new scale of coordinates for the baseline set computed at S50. *̅Z̅*̅₂̅ denotes the mean of the new scale of coordinates for the follow-up set computed at S50.

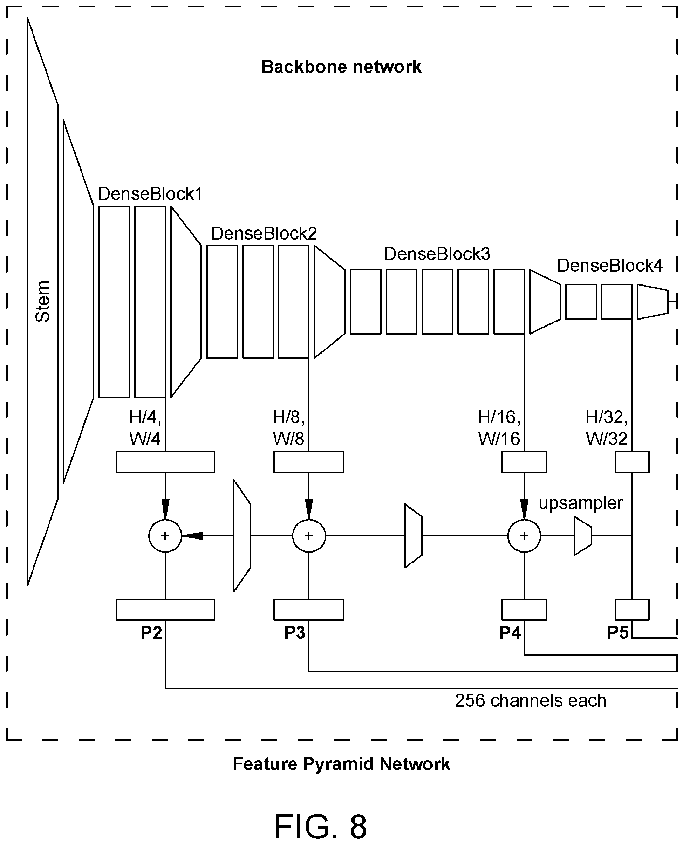

FIG. 8 illustrates the subtraction of the means. In other words, the difference between mean coordinates for the overlapping coordinates.

The method comprises aligning S70 the baseline set and the follow-up set by offsetting the images of the follow-up set with the obtained vertical offset. In other words, the aligning S70 adjusts the spatial displacement along the common reference axis between corresponding images of the baseline set and the follow-up set. For example, the set of coordinates of the images of the follow-up set may be subtracted with the vertical offset.

The computation of the mean for each set and the subtraction enables the method to correct for differences in the positioning of the images of the baseline set and the follow-up set, so that the alignment S70 ensures that the aligned baseline set and follow-up set represent the same part of the human body, thereby enabling an improved visual comparison of the change in a tissue of the human body (e.g., a lesion) represented in images of the follow-up set relative to the images of the baseline set. Consequently, the alignment performed by the method compensates the differences in alignment due to, e.g., patient positioning, differences in the acquisition set up such as acquisition angles or different imaging modalities.

In examples, computing the set of coordinates may comprise obtaining another (independent from the neural network trained for outputting the segmentation) trained neural network. This means that the computerized system performing the method can access directly the other neural network (e.g. the neural network runs onto the computerized system or is executed by the computerized system) or indirectly the other neural network (e.g. the computerized system can provide an input to the other neural network and obtain the output of the other neural network obtained for the input).

The other neural network may be configured to take as input sets of images, for example the baseline set or the follow-up set. The other neural network may also be configured to output a set of coordinates lying in a scale common to the pair of sets. In other words, the neural network may be configured for computing a location (determined by the coordinates) of the pair of sets based on the scale common to the pair. The scale may define the distance between each coordinate in the common reference axis. The other neural network may be a function comprising a collection of connected nodes, also called "neurons". Each neuron receives an input and outputs a result to other neurons connected to it. The neurons and the connections linking each of them have weight values, which are adjusted via a training.

Computing the set of coordinates may also comprise inputting the baseline set and the follow-up set to the trained other neural network. Computing S20 the set of coordinates may also comprise outputting the set of coordinates from the other neural network. The set comprises vectors of coordinates, for the baseline set and the follow-up set, lying in the scale common to the baseline set and the follow-up set. In other words, for each image of a respective set, the neural network outputs a coordinate associated to the slice lying in the scale common to the baseline set and the follow-up set. The result for a respective set is then a vector of coordinates.

The trained other neural network may have been trained according to a machine-learning method. In the context of the present disclosure, by "trained neural network", it is meant that weights of the neural network are definitively saved after performing a learning/training on a dataset.

The machine-learning method may comprise providing a dataset. The dataset may comprise sets of images, e.g., sets of medical images such as CT-Scans. The machine-learning method may also comprise training the other neural network based on the dataset. The training may comprise providing as input a set of images from the dataset and regressing a slice score for each image. In examples, the regression may be performed via backpropagation.

In examples, the training may be performed by:
- providing the dataset of sets images. Each set may comprise CT-scans, e.g., representing a desired anatomical coverage of the lesion;
- uniformly choosing a set i, a starting image *j* (from the set i), and a step between images k;
- sampling the chosen set i by selecting an image each *k* images (e.g., the image with index *j +k* and an image *j+* 2*k*), starting with the slice with index j, until arriving to the desired number of images in each set, denoted as *n_slices_per_sample;* and
- feeding the sampled images to the other neural network to regress slice scores, one for each image.

The other neural network may comprise a backbone responsible for extracting semantically relevant features maps, and additional head layers allow the regression of slice scores. The images may be input to the neural network via convolutional blocks. The neural network may also comprise additional layers such as convolutional layers, ReLU, Global Average Pooling and Fully Connected (FC).The layers output slice scores which are minimized with a loss, as explained below.

Preferably, the scores are regularly spaced, and correctly ordered as slices are also sampled regularly.

Regressing the slice score may comprise minimizing a loss. The loss may reward, for each slice score of a respective image of a set, at least one of an ordering of each slice score (e.g., denoted as an order loss term), attributing a regular spacing between pairs of slice scores (e.g., denoted as a distance loss term), and/or resembling to straight lines (e.g., denoted as an R2 loss term).

By rewarding an ordering of each slice score, it is meant that the loss penalizes the neural network for not ordering images correctly. In other words, the loss penalizes that a higher image (in the order defined by the its respective set) received a lower score than a lower image. By rewarding a regular spacing between pairs of slice scores, it is meant that the loss penalizes the network for not attributing regularly spaced slice scores, that is, for two consecutive images in the set, the difference in slice score should be uniform. By rewarding resembling to straight lines, it is meant that the neural network penalizes slice scores output by the neural network that do not fit a straight line.

The trained neural network thus outputs a common axial scale of normalized coordinates (in the form of the set of coordinates) for which a specific range of coordinates always refer to the same anatomical area.

The method thus relies on the alignment so as to track lesions. Lesion tracking enabled by the method uses information on lesions represented in the baseline set to infer the evolution of such lesions in the follow-up set. Thus, the method enables to detect and spot newly appeared lesion or disappeared lesion. By keeping an accurate track of lesions on a patient, medical practitioner are provided with a tool that allows assessing the disease progression over time.

Examples of the invention are now discussed with reference to FIG.s 3 to 7.

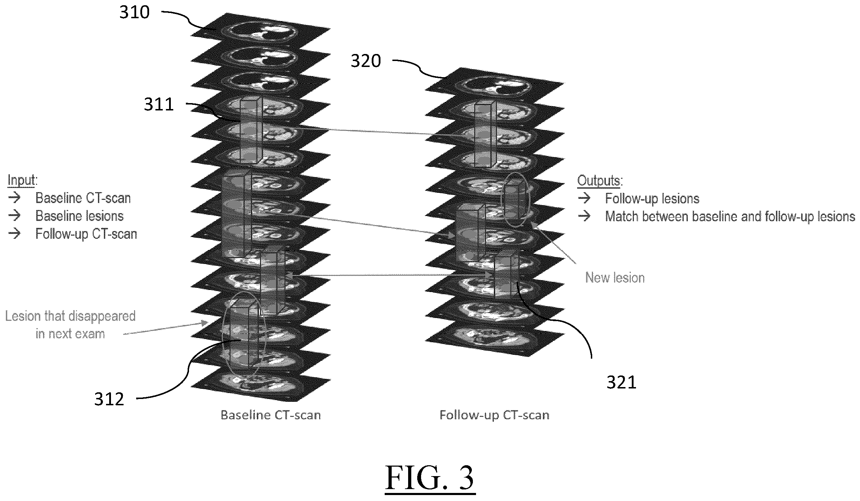

FIG. 3 illustrates the principle for following-up lesions.

The method takes as input a baseline set of CT-Scans 310. The baseline set represents also lesions (referred to as "baseline lesions"). The method also takes as input a follow-up scan 320. The baseline set comprises annotations related to the lesion represented by the images of the set associated to bounding boxes 311, 312. The output of the method are one or more bounding boxes 321 enclosing lesions of the follow-up set that relate to the baseline set. In other words, the bounding boxes 321 are the result of applying S40 the obtained transformation on annotations. Examples of the method may comprise using a segmentation neural network (LDA) on forced Regions of Interest (Rols). The output of the segmentation network results in aggregated 3D lesions in follow-up CT-scan.

The method also performs direct inference of lesions with the help of LDA on follow-up CT-scan.

The method also comprises performing a matching algorithm that compares propagated lesions to directly inferred lesions in the follow-up set and thus allows spotting newly appeared lesions.

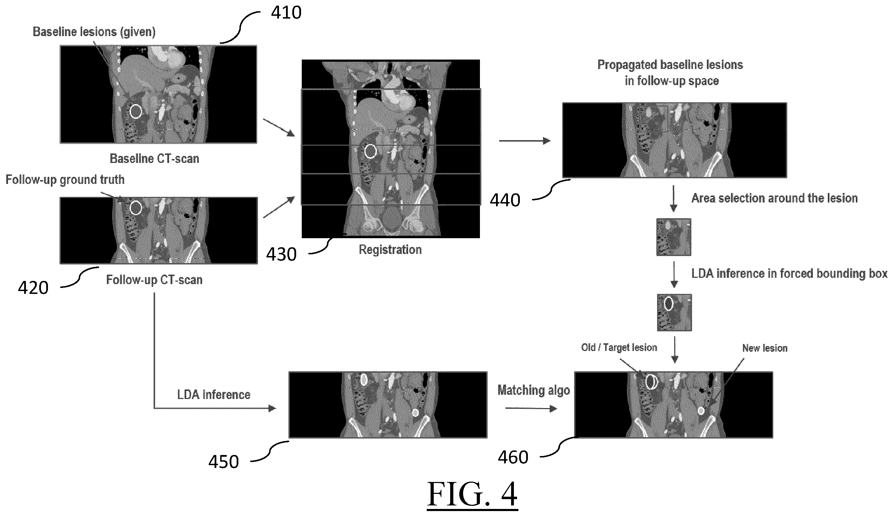

FIG. 4 illustrates an example of a pipeline 400 for implementing examples of the present method. In the pipeline 400, the method may comprise obtaining S10 the baseline set 410 and obtaining the follow-up set 420. The method may comprise performing S30 the registration of the baseline set and the follow-up set 430. The method may comprise applying S40 the obtained transformation on the at least one annotation, thereby obtaining the transformed bounding box in the follow-up set as a propagated lesion 440. The method may comprise determining S50 images 450 in the follow-up set 420 enclosed by the transformed bounding box and segmenting a lesion from the determined images. The lesion in the determined images enclosed by the transformed bounding box represents the tracked lesion 460 from the baseline set.

The method may apply a registration algorithm based on SimplelTK. SimplelTK is a simplified python interface to the Insight Segmentation and Registration Toolkit (ITK), which is an open-source library for performing medical image analysis. The use of the SimplelTK by the method allows using different optimization algorithms for aligning several medical data. The method uses a rigid transformation, more precisely an affine transformation that applies a translation, scaling and rotation.

Image registration is highly sensible to parameters initialization. A common practice consists in aligning volume centers, but it can be inaccurate as soon as the two volumes do not represent the same anatomical area or do not overlap sufficiently.

To solve these issues, the method performs the registration. The registration is initialized by inferring axial coordinates for both the baseline and the follow-up set based on a trained neural network. The architecture is based on a Z Coordinate Inference (ZCI) model, configured to projects slices into an axial scale common to both sets, such that close coordinates reflect the same anatomical area.

The registration computes a vertical offset based on the overlapping zone between volumes: it allows aligning them vertically. The resulting transformation matrix is initialized with the following parameters: $init transform = \left(\begin{matrix}{α}_{x} & 0 & 0 \\ 0 & {α}_{y} & 0 \\ 0 & 0 & {α}_{z}\end{matrix}\right) . \left(\begin{matrix}x \\ y \\ z\end{matrix}\right) + \left(\begin{matrix}{C}_{x} \\ {C}_{y} \\ Δ z\end{matrix}\right) = A . X + T$

***αₓ, α_{y}, α_{z}*** are dilations factors and correspond to the pixel spacing ratios between both CT-scan along each dimension (Vd ∈ [*x,y,z*]*,* ${α}_{i} = \frac{pixel {spacing}_{BL , d}}{pixel {spacing}_{FU , d}}$), C*ₓ,* C*_{y}* are the 2D translation components to align centers of volumes and Δz is the vertical translation offset.

The method also comprises optimizing the above transformation matrix based on a gradient descent algorithm with Mutual Information as a cost function.

The application S40 of the obtained transformation on the at least one annotation is now discussed.

Once the registration transformation is computed, it is possible to warp lesion information from baseline to follow-up space.

For each baseline lesion, the 3D bounding box coordinates are transformed to lie in the follow-up space. Due to rotation, it is possible that the resulting bounding box is not straight, so it is straightened by taking extremal coordinates of the warped 3D bounding box along each dimension. 3D warped bounding box z coordinates (*zₘᵢₙ*, *zₘₐₓ*) define the range of slices that are related to the baseline lesion in the follow-up volume, i.e. all slices for which the z coordinate is comprised in the interval [*zₘᵢₙ*, *zₘₐₓ*] is a candidate slice where to look for a lesion.

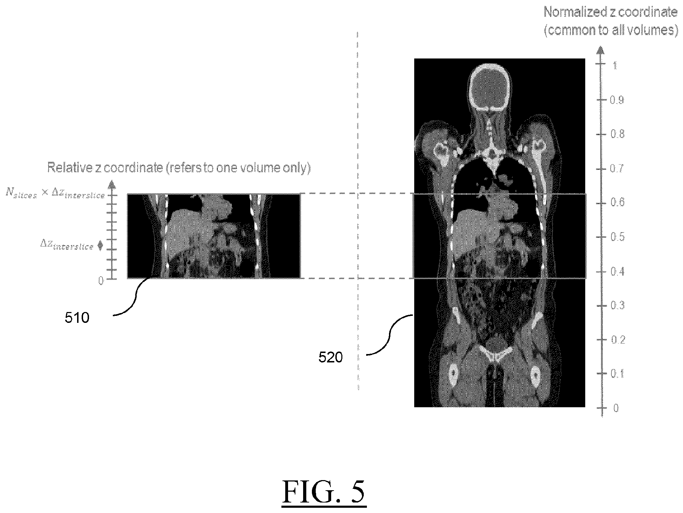

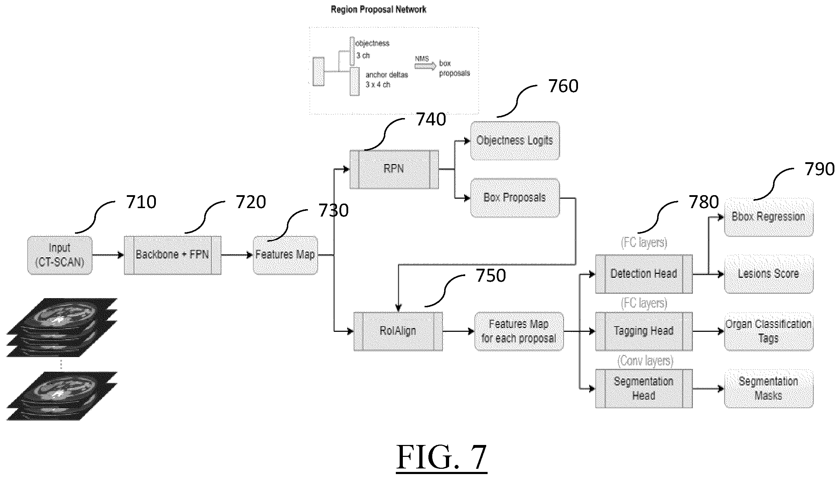

FIG. 5 illustrates an example of the Medical CT-scan relative vertical scale 510 vs. normalized vertical scale 520 obtained with the neural network, e.g. the neural network of FIG. 7.

For each related slice, the 2D projection of the 3D bounding box enlarged with the radius of the baseline lesion defines the searching area for further segmentation. The method enlarges the bounding box because the lesion has possibly moved in the body or changed in size under treatment. In addition, it can compensate some registration errors. The radius of the baseline lesion is arbitrary and is used to adapt the target bounding box size to the lesion size: it considers that the lesion cannot grow more than twice its original size between baseline and follow-up exam, or that it cannot move more than its radius inside the body.

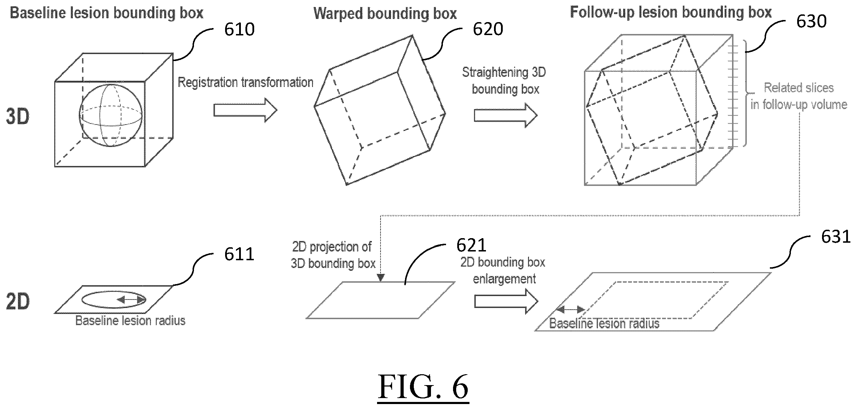

FIG. 6 illustrates an example of the application of the transformation on the at least one annotation associated to a bounding box on the baseline set, thereby propagating bounding boxes on the follow-up set. The method may start with a 3D bounding box 610 or a 2D bounding box 611 associated with an annotation obtained at S10. After applying the transformation, the method may comprise adjusting the position or orientation of the bounding box 620, 621 on the space associated with the follow-up set aligned with the baseline set. The method may comprise increasing the dimension of the bounding box 631, 630.

Now that the searching zone is well defined, the method infers lesions via a trained neural network, denoted as LDA model (Lesion Detection Assessment), which is configured to perform segmentation tasks for lesion detection.

FIG. 7 illustrates an example of an architecture of the neural network. The neural network receives as input an image, such as a CT-SCAN 710. The neural network also comprises a Backbone Layer and a Feature Pyramid Network (FPN) Layer 720. The output is a feature map 730. The feature map 730 is input to a Region Proposal Network 740 and to a Region of Interest alignment network (RolAlign) 750. The output of the RPN 760 may be send to RoiAlign 750. The network also comprises a detection head, tagging head and segmentation head 780. The network outputs a bounding box regression, a lesion score, an organ classification tag and segmentation masks 790.

In the example, the Region Proposal Network (RPN) is not applied since the bounding boxes in which baseline lesions should be found are known. Therefore, the segmentation network is applied. If a lesion is detected inside the provided bounding box, the method obtains a segmentation mask. Otherwise, it means that the lesion is not present on the studied slice. If none of the baseline lesion related slices in follow-up volume yields a segmentation mask, it means that the lesion has disappeared.

FIG. 8 illustrates the Backbone Network and the Feature Pyramid Network.

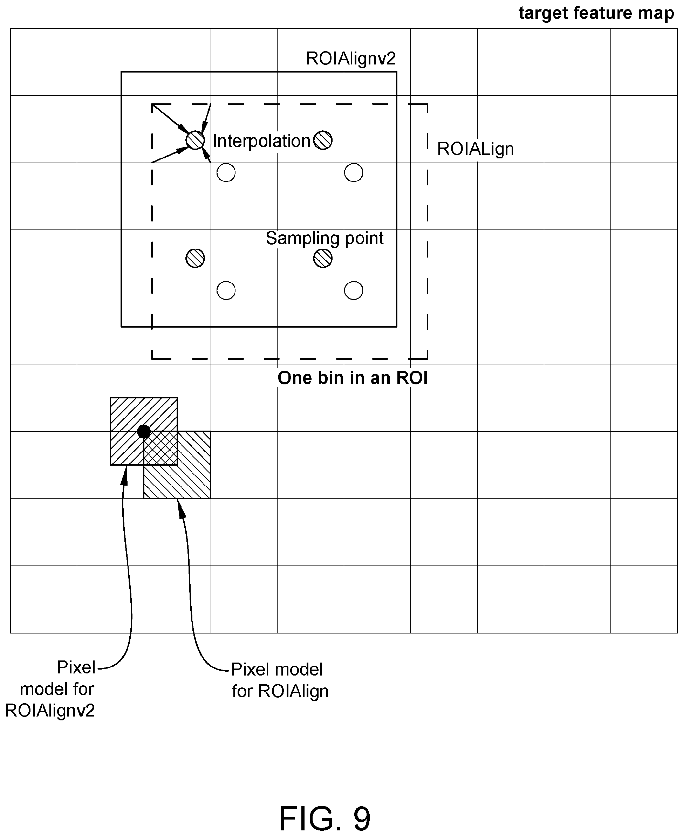

FIG. 9 illustrates the output of RoiAlign.

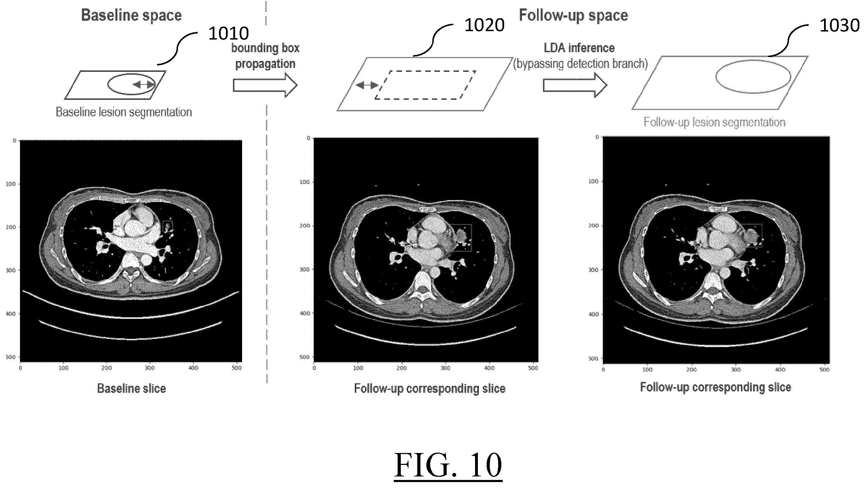

FIG. 10 illustrates an example of the propagation of bounding boxes and segmentation of the lesion in an image of the follow-up set. The image of the baseline 1010 represents a lesion enclosed by a bounding box. After transformation, the method finds a bounding box 1020 on the follow-up image. The method comprises obtaining a segmentation 1030 of the lesion inside the bounding box on the follow-up image.

The LDA aggregation algorithm described in the European patent application EP23307178.6 may be used for enhancing the resulting 2D segmentations to unique non-overlapping 3D lesions.

The method may comprise applying an overlapping region-based matching algorithm to compare the list of propagated lesions from baseline to follow-up volume to the list of directly inferred lesions on follow-up volume: this enables detecting new lesions that have appeared between baseline and follow-up exam.

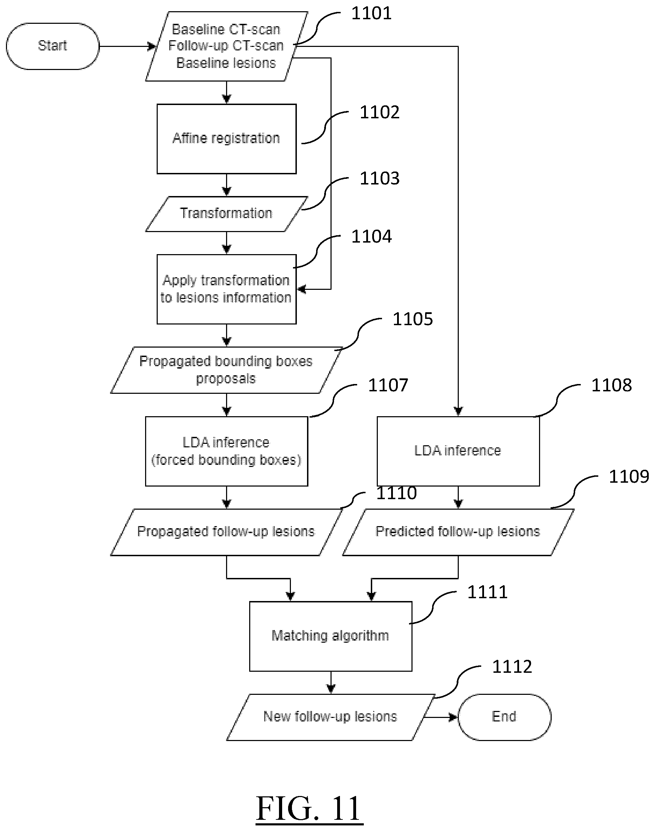

FIG. 11 illustrates a flowchart of an example of the method of the present invention.

At 1101, the flowchart comprises obtaining a baseline set of images, and at least one annotation related to the lesion represented by at least one image of the set, the at least one annotation being associated to a bounding box.

At 1102, the method comprises performing a registration (affine registration) of the baseline set and the follow-up set, the registration thereby obtaining a transformation at 1103 that aligns the baseline set and the follow-up set in space;

At 1104, the method comprises applying the obtained transformation on the at least one annotation, thereby obtaining the transformed bounding box (i.e., propagated bounding boxes proposals 1105) in the follow-up set.

The method determines images in the follow-up set enclosed by the transformed bounding box and segmenting a lesion from the determined images, the lesion in the determined images enclosed by the transformed bounding box representing the tracked lesion from the baseline set. This may be performed by using an LDA inference 1108, together with an LDA inference 1107 with forced bounding boxes on the proposals 1105, while using a matching algorithm 1111 for determining the follow-up lesions 1112.

## Claims

1. A computer implemented method for tracking a lesion of a patient from at least two sets of images representing the lesion, each set of images being acquired at different time points, comprising:
- obtaining (S10) a baseline set of images, and at least one annotation related to the lesion represented by at least one image of the set, the at least one annotation being associated to a bounding box;
- obtaining (S20) a follow-up set of images;
- performing (S30) a registration of the baseline set and the follow-up set, the registration thereby obtaining a transformation that aligns the baseline set and the follow-up set in space;
- applying (S40) the obtained transformation on the at least one annotation, thereby obtaining the transformed bounding box in the follow-up set;
- determining (S50) images in the follow-up set enclosed by the transformed bounding box and segmenting a lesion from the determined images, the lesion in the determined images enclosed by the transformed bounding box representing the tracked lesion from the baseline set.

2. The method of claim 1, further comprising, after applying (S40) the obtained transformation and prior to determining images in the follow-up set enclosed by the transformed bounding box and segmenting the lesion in the determined images, increasing the dimension of the bounding box.

3. The method of claim 1 or 2, wherein the obtained transformation is one of an affine transformation or a deformable registration.

4. The method of claim 3, wherein the obtained transformation is an affine transformation of the type: $transform = \left(\begin{matrix}{α}_{x} & {r}_{xy} & {r}_{xz} \\ {r}_{yx} & {α}_{y} & {r}_{yz} \\ {r}_{zx} & {r}_{zy} & {α}_{z}\end{matrix}\right) . \left(\begin{matrix}x \\ y \\ z\end{matrix}\right) + \left(\begin{matrix}{C}_{x} \\ {C}_{y} \\ Δ z\end{matrix}\right) = A . X + T$ wherein ***αₓ, α_{y}, α_{z}*** denote dilation factors in [*x, y, z*] coordinates, *r_{xy}, r_{xz}, r_{yx}, r_{yz}, r_{zx}, r_{zy} denote rotation coef ficients,* C*ₓ,* C*_{y}* denote translation components in the x and y coordinates, and Δz denotes a vertical translation offset in z coordinates.

5. The method of any one of claims 1 to 4, wherein:
- the bounding box is a 2D bounding box, and segmenting the lesion in the determined images comprises obtaining a neural network configured to take as input a set of images and to output a 2D segmentation of a lesion from the set of images, and applying the neural network to the determined images; or
- the bounding box is a 3D bounding box, and segmenting the lesion in the determined images comprises obtaining a 3D segmentation of the lesion.

6. The method of any one of claims 1 to 5, further comprising inferring a new lesion in the follow-up set by:
- performing a segmentation of the baseline set of images; and
- comparing the segmentation in the determined images with the segmentation of the baseline set of images.

7. The method of claim 6, wherein performing the segmentation of the baseline set of images comprises applying a trained segmentation neural network to the baseline set, the trained segmentation neural network being configured to take as input one or more images and at least one annotation associated to a bounding box and to output a pair of a respective segmentation of a lesion and a respective bounding box enclosing the lesion.

8. The method of any one of claims 1 to 7, wherein performing (S30) the registration of the baseline set and the follow-up set comprises aligning the baseline set and the follow-up set along a common reference axis, the common reference axis being normal to each image of the baseline set and the follow-up set, the alignment being performed by:
- obtaining a predetermined interslice space for each of the baseline set and the follow-up set;
- computing, for each set, a set of coordinates lying in a scale common to the baseline set and the follow-up set each coordinate being associated, in the common reference axis, with an image;
- computing an intersection between the two sets of coordinates, thereby identifying overlapping images, which having coordinates which lie in the intersection;
- creating, for each set, a new physical scale of coordinates sharing a common origin, and wherein two consecutive coordinates of the new physical scale being are spaced by the predetermined interslice space;
- computing, for each set, the mean of the new physical scale of coordinates associated to overlapping slices;
- subtracting the mean computed for the follow-up set to the mean computed for the baseline set, thereby obtaining a vertical offset along the common reference axis; and
- aligning the baseline set and the follow-up set by offsetting the follow up set with the obtained vertical offset.

9. The method of claim 8, wherein computing the set of coordinates comprises:
- providing another neural network, the other neural network being configured to take as input a pair of sets of images and to output a set of coordinates lying in a scale common to the pair of sets, each coordinate being associated, in the common reference axis, with an image;
- inputting the baseline set and the follow-up set to the other neural network; and
- outputting the set of coordinates from the other neural network.

10. The method of claim 9, wherein the other neural network is trained according to a machine-learning method comprising:
- providing a dataset of sets of images; and
- training the other neural network based on the dataset, the training comprising providing as input a pair of sets of images from the dataset and regressing a slice score for each image of the set.

11. The method of any one of claims 1 to 10, wherein each image is a CT-Scan.

12. The method of any one of claims 1 to 11, further comprising displaying and/or storing the aligned baseline set and follow-up set of images.

13. A computer program comprising instructions for performing the method of any of claims 1 to 12.

14. A computer readable storage medium having recorded thereon a computer program of claim 13.

15. A system comprising a processor coupled to a memory, the memory having recorded thereon the computer program of claim 13.
